Europäisches Patentamt

European Patent Office

Office européen des brevets

(19)

(11) Publication number: **0 136 781**
A2

(12) # EUROPEAN PATENT APPLICATION

(21) Application number: **84305204.4**

(22) Date of filing: **31.07.84**

(51) Int. Cl.⁴: **A 61 K 37/24**
**A 61 K 9/00**
**//(A61K37/24, 37:02)**

(30) Priority: **09.08.83 US 521786**

(43) Date of publication of application:
**10.04.85 Bulletin 85/15**

(84) Designated Contracting States:
**AT CH FR GB LI LU NL SE**

(71) Applicant: **AMERICAN HOME PRODUCTS CORPORATION**
**685, Third Avenue**
**New York, New York 10017(US)**

(72) Inventor: **Jones, Robert Clyde**
**117 North Essex Avenue**
**Narberth Pennsylvania(US)**

(74) Representative: **Brown, Keith John Symons et al,**
**c/o John Wyeth & Brother Limited Patent and Trademark**
**Department Huntercombe Lane South**
**Taplow Maidenhead Berkshire SL6 0PH.(GB)**

(54) **Treatment of endometriosis.**

(57) Endometriosis may be treated with LHRH or an agonist thereof to create a hypoestrogenic state and a progestational agent to cause decidualization, fibrosis, necrosis and absorption of the implant. Compositions and packages for use in treating endometriosis contain LHRH or an agonist thereof and a progestational agent.

EP 0 136 781 A2

External endometriosis is a condition in which endometrial tissue is found outside of the uterus. Common sites of endometriotic loci are: ovaries, uterine ligament, rectovaginal septum, pelvic peritoneum, umbilicus, laparotomy scars, hernial sac, appendix, vagina, vulva and cervix. Symptoms of endometriosis include dysmenorrhea, menorrhagia, pelvic pain, irregular cycles and infertility. Radical surgery, including removal of the ovaries to eliminate the source of gonadal steroids, may be recommended in extreme cases of this disease. A more conservative approach to the treatment of endometriosis is hormonal therapy.

Hormonal management of endometriosis generally revolves around two therapies. One regimen involves the use of œstrogen-progestogen combinations to produce a state of pseudopregnancy, while the second method involves the use of danazol to produce a pseudomenopausal state. Both regimens are used extensively where future child-bearing is a consideration.

The concept of inducing a state of pseudopregnancy to treat endometriosis is based on the observation that the disorder occasionally regresses during pregnancy [J. V. Miegs, Boston M & S Journal, 187, 1 (1922); J. A. Sampson, Surg. Gynec. & Obst., 38, 287 (1924); C. T. Beecham, J.A.M.A., 139, 971 (1949)]. During pregnancy, oestrogen and progesterone levels are elevated, ovulation is suppressed and amenorrhea is induced. In an analogous manner, the use of oral contraceptives produces a pseudopregnancy in which the hormonal pattern mimics that of pregnancy with the induction of amenorrhea and anovulation. As a consequence, decidualization of the endometrial foci occurs with subsequent necrosis and fibrosis. A state of amenorrhea is important in that it prevents re-seeding of endometrial cells (via retrograde menstrual flow) to an extra-uterine site. However, the untoward side effects of combination oral contraceptives, such as, nausea, cramps, weight gain, acne, etc. may militate against this course of therapy for many women.

During menopause, a period of low oestrogen and progesterone secretation, anovulation and amenorrhea, endometriosis generally regresses [A. A. Luciano, Contemporary Ob./Gyn., 19, 211 (1982)]. On the belief that the drug, danazol, inhibits gonadotrophin release and, consequently, sex steroid secretion, with concomitant amenorrhea, this steroid was employed to produce a pseudomenopausal state [Greenblatt et al., Fertil Steril., 22, 102, (1971)].

Although danazol produces amenorrhea and anovulation, its mode of action is not entirely understood. It has been suggested that danazol inhibits development of the follicle directly to produce anovulation during the course of treatment [Luciano et al., Am. J. Obstet. Gynecol., 141, 723 (1981)]. Also, androgens cause atrophy of normal endometrium and endometriomatas [Creadick, N.C. Med J., 11, 576 (1950)], and danazol, by virtue of its slight androgenicity, may cause regression of the endometriotic foci directly.

The adverse side-effects of the use of danazol include weight gain, edema, decrease in breast size, acne, hirsutism, oily skin, deepening of the voice and elevations of both serum glutamic oxaloacetic transmainase (SGOT) and serum glutamic pyruvic transaminase (SGPT) [Barbieri et al., Fertil Steril., 37, 737 (1982)]. The elevation of SGOT and SGPT levels and fluid retention contraindicates the use of danazol in patients with liver disease and cardiac or renal diseases. In addition, pregnancy must be ruled out absolutely since danazol can cross the placenta and cause pseudohermaphroditism [Duck and Katayama, Fertil Steril., 35, 230, (1981)].

- 4 -

In addition to the numerous side-effects associated with both courses of treatment and the prohibitive cost entailed through the use of danazol, neither combination oral contraceptives nor danazol causes total remission of endometriosis. Thus, a critical need exists for a therapy that has minimal side-effects, causes remission (preferably total) of the disease and is relatively inexpensive.

A new approach to the treatment of endometriosis is the use of LHRH agonists [Meldrum et al., J Clin Endocrinol Metab, 54, 1081, 1982a; Meldrum et al., 64th Annual Meeting of the Endocrine Society 1982b (Abstract 665); Lemay and Quesnel, Fertil Steril., 38, 376 (1982)]. Essentially, these agonists produce downregulation and desensitization of the pituitary-ovarian axis, and consequent "medical oophorectomy" associated with hypoestrogenic state. However, the external endometriotic tissue resumes growth once treatment with LHRH or an agonist thereof is withdrawn. This return of growth after withdrawal of LHRH or agonist treatment is akin to the loss of dormancy observed in endometrial tissue of patients treated with an oestrogen even after being ovariectomized. Thus, although a dormant state devoid of signs or symptomology of endometriosis can be established by removal of the ovaries or treatment with LHRH or an agonist thereof, that disease-arrested state reverses upon re-establishment of endogenously produced or oxogenously introduced oestrogen levels.

Thus, the nature of extra-uterine tissue is not altered by treatment of the patient with danazol, LHRH or agonists thereof or oestrogen-progestogen combination therapy (oral contraceptives producing a pseudopregnancy), although the functionality of such tissue is diminished during chronic treatment with all these therapeutics.

Auclair et al, Archives of Andrology, $\underline{8}$, 21 (1982) discloses the possible treatment of prostatic cancer with an LHRH analogue [(D-Ala$^6$.des-Gly-NH$_2$$^{10}$)LHRH ethylamide] and medrogestone, a compound with progestational and antiandrogenic properties.

In accordance with this invention there is provided a method and pharmaceutical composition for treatment of endometriosis.

The invention particularly provides a composition of matter for the treatment of endometriosis in a female placental mammal comprising LHRH or an agonist thereof and a progestational agent, with the proviso that the progestational agent is other than medrogestone.

The LHRH or an agonist thereof in the pharmaceutical composition may be in an amount sufficient to produce a hypoestrogenic state in the female mammal. The progestational agent in the pharmaceutical composition may be in an amount to decidualize endometrial tissue. Optionally one or more pharmaceutically acceptable adjuvants and fillers may be incorporated in the composition.

The invention also provides a package for simultaneous or sequential use in the treatment of endometriosis in a female placental mammal which comprises a first pharmaceutical composition comprising LHRH or an agonist thereof and a pharmaceutically acceptable carrier and a second pharmaceutical composition comprising a progestional agent and a pharmaceutically acceptable carrier, with the proviso that the progestational agent is other than medrogestone. The amounts of LHRH or agonist thereof and progestational agent may be as mentioned hereinabove.

Preferably, one of the "super" agonists of LHRH known to the art, such as $D-Trp^6-DesGly^{10}-Pro^9-NHEt-LHRH$, $D-Trp^6-N^\alpha-MeLeu^7-Des-Gly^{10}-Pro^9-NHEt-LHRH$, $D-Ser(t-butyl)^6-DesGLy^{10}-Pro^9-NHEt-LHRH$, and the like is employed as the LHRH agonist (e.g. to produce the hypoestrogenic state by inhibition of ovarian steroid production). Preferably levonorgestrel is employed as the progestational agent. Levonorgestrel may act directly on endometrial explants to decidualize the tissue, induce a "burned-out" effect and necrosis known to attend long term progestational treatment. In this manner, the combination therapy produces a more beneficial effect than either agent produces alone.

Examples of progestational agents which may be effectively employed in the compositions of this invention are levonorgestrel, desogestrel, or medroxyprogesterone acetate. These agents are not inherently oestrogenic nor are they converted to oestrogens upon oral or buccal administration. Levonorgestrel is the preferred progestational agent.

The invention further provides the use in the treatment of endometriosis in a female placental mammal of LHRH or an agonist thereof in an amount and for a time sufficient to establish a hypoestrogenic state, and of a progestational agent administered during the hypoestrogenic state in an amount and for a time to produce an atrophic nonendometrial implant. The treatment of endometriosis in accordance with this invention can comprise administering LHRH or an agonist thereof to a female placental mammal in need of such treatment, initially either alone or in conjunction with a progestational agent for a time and in an amount sufficient to create a hypoestrogenic state which evidences diminished secretation of steroids from the ovaries, at which time treatment with the progestational agent, alone or in combination with LHRH or an agonist thereof is initiated if it was not employed initially. The progestational agent acts directly on the endometrial tissue. Prolonged stimulation by the progestational agent produces a decidual reaction and ultimately necrobiosis and complete absorption of the implant.

The effectiveness of the method of this invention and the pharmaceutical composition when used in that method has been established by the following procedure. The nonapeptide selected as representative of LHRH and agonists thereof is D-Trp$^6$-N$^\alpha$-MeLeu$^7$-Des-Gly$^{10}$-Pro$^9$-NHEt-LHRH hereinafter referred to as Agonist A.

### Materials and Methods

Adult Charles River Sprague-Dawley CD female rats (200-240g) were used. The animals were anesthetized with diethyl ether and a 5 cm incision, oblique (right) to the midline, was made from a point proximal to the position of the urethra upward. A second 4 cm oblique incision was made through the musculature of the body wall. The uterine blood vessels were ligated and an 8 mm segment of the right uterine horn was excised. The uterine segment was cut longitudinally, and the myometrium removed. A 5 x 5 mm section of the endometrium was transplanted to the peritoneal cavity with the epithilial lining of the uterine segment opposed to the body wall. The explanted endometrial tissue was sutured at its four corners to the body wall using 0.5 metric green polyester braid (Ethiflex, 7-0). The stage of the estrous cycle was determined at the time of operation.

Within 3 weeks, growth of the explant was at its maximum. The criterion of a viable graft is the accumulation of fluid similar to that which occurs in the uterus as a result of oestrogen stimulation.

- 8 -

The animals were injected subcutaneously (s.c.) for 42 days with 100 ug of Agonist A or levonorgestrel per day. A third group received 50 ug of Agonist A plus 50 ug levonorgestrel in separate injections for the same period of time. At the end of the 6 week treatment period, the animals were laparotomized and the condition of the explant examined. Eight weeks after cessation of treatment the animals were again laparotomized to determine the condition of the explant. The animals were autopsied 12 weeks after cessation of treatment and the explant was preserved in 10% formalin.

Two end-points of the study are (1) regression of growth after 42 days of treatment, (2) recurrence of growth at 8 and 12 weeks after cessation of treatment. Subjectively, four classifications of regrowth are as follows:

    None - explant visually absent
    Minimal (Min) - explant present but no fluid
    Moderate (Mod) - some fluid present in explant
    Excellent - growth approximates that prior to
                                              treatment
            (3 weeks post-explanation)

The results obtained with a particular course of treatment were detailedfor each individual rat in the following tables.

## Results

The effects of all compounds on experimental endometriosis are summarized in Tables I, II and III.

## TABLE I

### Subjective Evaluation of Effect on Endometrial Explant - Agonist A

### Condition (Growth) of Explant

| Rat | Treatment (Rx) | [a] Pre-Rx (3 weeks after operation) | [b] After 6 weeks Rx | [c] 8 weeks after cessation of Rx | [d] 12 weeks after cessation of Rx |
|-----|----------------|------------------------------------|---------------------|-----------------------------------|------------------------------------|
| 1. | Agonist A 100µg | Good | Complete regression* | ------- | ------ |
| 2. | Agonist A 100µg | Excellent | Complete regression | Excellent; larger * than original explant | ------ |
| 3. | Agonist A 100µg | Excellent, large | Almost complete regression; fluid presence questionable | Poor; 1/4 size of original explant; yellow | Almost gone; 1/5 size of original explant * |
| 4. | Agonist A 100µg | Good | Complete regression | Excellent; smaller than original explant | Almost gone; 1/5 size of original explant * |
| 5. | Agonist A 100µg | None; explant visually present | Explant visually present | Excellent; large growth | Excellent; very large * |
| 6. | Agonist A 100µg | Excellent; large | Moderate regression; 2/3 size of original explant | Complete regression; explant visually present | * Complete regression explant visually present |

* Autopsy

a) Five of the explants exhibited good to excellent growth. The remaining explant (No. 5) was visually present but was devoid of fluid.

b) Complete regression of the explant was observed in 3 rats. In two other rats moderate regression of the explant was exhibited in one while a minimal to questionable amount of fluid was present in the second explant. The explant which exhibited no growth prior to treatment (No. 5) was visually present.

c) Five rats were examined for renewal of growth; two exhibited excellent growth of the explant, one moderate and one complete regression of growth. The one explant which was only visually present after six weeks of treatment (No. 5) exhibited excellent growth.

d) Two of the rats examined exhibited almost complete regression of growth and in one rat the explant was only visually present. The final rat in which only the explant itself was visible (No. 5) prior to treatment exhibited extreme fluid distension.

# TABLE II

## Subjective Evaluation of Effect on Endometrial Explant - Levonorgestrel

### Condition (Growth) of Explant

| Rat | Treatment (Rx) | [a] Pre-Rx (3 weeks after operation) | [b] After 6 weeks Rx | [c] 8 weeks after cessation of Rx | [d] 12 weeks after cessation of Rx |
|---|---|---|---|---|---|
| 1. | Levonorgestrel 100μg | Excellent | Excellent-no change* | ------- | ------- |
| 2. | Levonorgestrel 100μg | Excellent | Excellent; slight regression; yellow | Excellent - same size as original explant | ------- |
| 3. | Levonorgestrel 100μg | Excellent-Large | Excellent; slight regression | Excellent; slightly smaller than original | Moderate; 2/3 size original explant; partially yellow * * |
| 4. | Levonorgestrel 100μg | Excellent-Large | Moderate; some regression | Excellent; slightly smaller than original | Moderate; 1/2 size original explant * * |
| 5. | Levonorgestrel 100μg | Excellent-Large | Almost complete regression | Moderate; 1/4 size of original explant | Poor; 1/5 size of original explant; partially yellow * |
| 6. | Levonorgestrel 100μg | Excellent-Large | Good regression; 1/3 size of original explant | Moderate; 1/3 size of original explant | Moderate; 1/5 size original explant * |

* Autopsy
a) All six rats exhibited well developed, fluid filled endometrial explants.
b) Four of the six explants exhibited only minimal or no regression of growth. In the remaining two rats, one explant had regressed almost completely, and the remaining explant was one-third of its original size.
c) Five rats were examined to determine the condition of the explant; three exhibited excellent growth of the explant and two moderate growth.
d) Three explants of the four rats examined exhibited poor growth; the remaining explant exhibited moderate growth.

TABLE III

Subjective Evaluation of Effect on Endometrial Explant - Levonorgestrel + Agonist A

## Condition (Growth) of Explant

| Rat | Treatment (Rx) | [a] Pre-Rx (3 weeks after operation) | [b] After 6 weeks Rx | [c] 8 weeks after cessation of Rx | [d] 12 weeks after cessation of Rx |
|---|---|---|---|---|---|
| 1. | Levonorgestrel 50µg + Agonist A 50µg | Excellent; large | Almost complete * regression; 1/5 size of original explant; hard; yellow | - - - - - - | - - - - - - |
| 2. | Levonorgestrel 50µg + Agonist A 50µg | None; explant visually present | Explant visually present | Good; hard * | - - - - - - |
| 3. | Levonorgestrel 50µg + Agonist A 50µg | Excellent; large | Excellent; slight regression; hard; partially yellow | Slight regression; 4/5 size of original explant; hard; partially yellow | Poor; 1/5 size of original explant * |
| 4. | Levonorgestrel 50µg + Agonist A 50µg | Excellent; large | Excellent; slight regression; yellow | Slight regression; 4/5 size of original explant; hard; yellow | Poor; regression; 1/5 size of original explant; hard; yellow * |
| 5. | Levonorgestrel 50µg + Agonist A 50µg | Good; small | No change in size; hard; hemorrhagic | Good; some regression; 2/3 size of original explant; hard; hemorrhagic | Poor; 1/5 size of explant; hard; hemorrhagic * |

0136781

TABLE III (cont.)

| Rat | Treatment (Rx) | [a] Pre-Rx (3 weeks after operation) | [b] After 6 weeks Rx | [c] 8 weeks after cessation of Rx | [d] 12 weeks after cessation of Rx |
|---|---|---|---|---|---|
| 6. | Levonorgestrel 50µg + Agonist A 50µg . | Good; small | Moderate; some regression; 2/3 size original explant | Excellent; larger than original explant; hard | Very poor; 1/5 size original explant; hard; partially yellow |

\*   Autopsy

a)   Five of the explants exhibited well developed growth; the remaining explant (No. 2) was visually present but was devoid of fluid.

b)   One explant exhibited almost complete regression while four explants showed only slight regression of growth. The remaining explant (No. 2), devoid of fluid prior to treatment, was still visually present.

c)   Slight regression of growth was exhibited in three rats, while another explant (No. 6) was larger than that prior to treatment. In addition, three of the above explants appeared to have assumed a tough fibrotic character rather than the resiliency usually associated with a fluid filled explant. The explant exhibiting no growth prior to treatment (No. 2) showed moderate growth and was fluid filled.

d)   All four explants exhibited good reduction in growth and also appeared to have assumed the property of hard, fibrotic cysts.

Following the same procedure, danazol was administered to a group of six rats at a rate of 30µg per day. After twenty-one days of treatment, no regression of the explant growth could be observed. Eight weeks after cessation of treatment, one rat showed excellent growth while four exhibited sub-minimal growth of the explant.

An additional group of five rats were ovariectomized. Removal of the ovaries produced complete regression of growth of the explant in all five rats within three weeks of the operation. However, the explant was still visually evident. The animals were autopsied eleven weeks (corresponding to the eight week drug-free period in dosed animals) after ovariectomy at which time remnants of the explant appeared to be present in all five rats. This demonstrates that growth of the explant is dependent upon ovarian steroids.

The administration of Agonist A produced a substantial decrease in size in all of the explants which had accumulated fluid prior to treatment. However, the resumption of growth observed at both 8 and 12 weeks after cessation of treatment indicated that the majority of explants were still viable. Generally, a reduction in size of the explants was observed 12 weeks after cessation of treatment; this suggests that the partial inhibition of growth may be permanent. However, with regard to suppression of growth during treatment and resumption of growth after cessation of treatment, the administration of 100µg Agonist A for 6 weeks afforded no advantage over treatment with 1 or 30µg for 3 weeks.

It would appear that in this animal model, increasing the amount of Agonist A above an optimum dose of 1-10 µg produced no further inhibition of explant growth. On the other hand, if the reduction in explant size observed 12 weeks after cessation of treatment is permanent, a larger injection period (3-6 months) might eliminate the explant completely.

The results obtained with levonorgestrel demonstrated that there was little affect on growth of the explant after 6 weeks of treatment or at 8 weeks after cessation of treatment. Although 100 µg of levonorgestrel blocks gonadotrophin release, this effect is masked by a direct stimulatory effect on the explant itself during the period of treatment and for some weeks thereafter. The reduction in size of the explant observed 12 weeks after cessation of treatment again demonstrated the potential of this steroid in the treatment of endometriosis, although its effect was delayed. However, as with the results obtained with Agonist A, the administration of 100 ug levonorgestrel for 6 weeks appeared to be no more efficacious than 100 µg for 3 weeks. It is possible, of course, that a greater reduction in explant size would have been observed had the recovery period been extended beyond 12 weeks.

Danazol did not inhibit growth of the endometrial explant during treatment at a daily dose of 30 ug for 3 weeks. However, 3 of the explants exhibited extremely minimal growth 8 weeks after cessation of treatment. These results suggest that danazol may have a direct stimulatory effect on the explant similar to that of levonorgestrel.

Removal of the ovaries effectively inhibited growth of the explant in all 5 rats 31 days after ovariectomy. However, at 11 weeks after ovariectomy, visual examination suggested that remnants of the explant were still present. While removal of the ovaries does not appear to eliminate the explant completely it effectively retards its growth sufficiently to provide a reasonable goal to be achieved by the various drug treatments.

The combination treatment employing Agonist A and levonorgestrel produced the most interesting results. Little inhibition of explant size was observed after 6 weeks of treatment and at 8 weeks after cessation of treatment; this lack of inhibition of growth was interpreted as being due to direct progestational stimulation of the explant by levonorgestrel. However, at these two periods of observation some of the explants acquired a yellow cast. The yellow colour was only observed in one rat which had received Agonist A (No. 3, 8 weeks after cessation of treatment) and in one rat injected with levonorgestrel (No. 5, 12 weeks after cessation of treatment). In addition, the resiliency of the explants in the rats receiving combination treatment diminished. At 12 weeks after cessation of treatment all of the explants had lost their flexibility, become hardened and taken on a yellow cyst-like appearance, characteristic of necrotic tissue being absorbed by the body. Although applicant does not want to be bound by any specific mode of action, it is believed that the combination therapy leads to a physiologic state in which the peptide, Agonist A, produces a rapid onset of a hypoestrogenic state by indirectly inhibiting steroid production in the ovary (chemical castration). Levonorgestrel, by virtue of its progestational and/or androgenic properties, acts directly on the explant to produce a decidualized endometrium. Constant stimulation of the endometrium by levonorgestrel produces necrosis of the explant leading to atresia or the "burned-out" effect. As such, the combination of Agonist A and levonorgestrel inhibits the viability and growth of the endometrial explant more effectively than either compound alone.

Of the two rats without fluid accumulation in the explant prior to the initiation of the study (No. 5 in Table I and No. 2 in Table III), one exhibited excellent growth after cessation of treatment with Agonist A alone and the other one, moderate growth after cessation of treatment with Agonist A in combination with levonorgestrel. The explant in the rat treated with the peptide was four times as large as the explant in the animal treated with the combination of the peptide and steroid. The growth of these explants after cessation of treatment may have resulted from a rebound effect. In short, once the inhibiting influence of Agonist A is removed the pituitary and ovary secrete, for a short period of time, more than normal amounts of gonadotrophins and steroids, respectively; steroid overstimulation of the explants may explain their growth. The moderate growth of the explant observed in the animal receiving combination treatment may have been due to the lower dose of Agonist A employed (50 µg) or to a residual effect of levonorgestrel.

In summary, this study demonstrates that Agonist A administered over a 6 week period is insufficient to eliminate the extra-uterine growth of endometrial tissue. However, the reduction in size of the explants 12 weeks after cessation of treatment suggests that the partial inhibition of growth may be permanent. Thus, a longer period of treatment may eventually destroy the viability of the explant. There was an increase in explant growth in a few rats after cessation of treatment that suggests that exacerbation of the disease may ensue after treatment is withdrawn. Levonorgestrel also inhibited growth of the explant. The reduction in explant size between 8 and 12 weeks after cessation of treatment demonstrates a delayed effect. A longer recovery period may be required to observe further inhibition of explant growth. Treatment with the combination of Agonist A and levonorgestrel inhibited growth and outwardly changed the character of the explant from flexible to hard.  The combination of LHRH or an agonist thereof and levonorgestrel clearly appears to be the route of choice in the treatment of endometriosis.

Thus, the combination therapy of this invention employing LHRH or an agonist thereof in conjunction with a progestational agent provides the only means for converting endometrial implants into tissue which is not stimulated by ovarian steroids as endometrial tissue.

In practice, the combination therapy of this invention is initiated in the endometriotic patient and progress is followed into the hypoestrogenic state via blood analysis.  After all symptoms of endometriosis have been relieved, the dosing regimen (either combination or progestational  agent alone) is continued and progress in the decidualization of endometrial implants followed by absorption of the fibriotic tissue may be followed by laparoscopic examination.  The term contemplated for overall therapy resulting in complete regression of an implant is minimally 30-45 days, with expected continuation for periods as long as one year or more.  Any such treatment term depends upon the severity of the disease and the individual patient's response to the therapy. Therefore, close observation of the progress of disease remission is required.

Initial dosing with LHRH or an agonist thereof in an amount of about three or more micrograms per kilogram patient weight, based upon the potency of the polypeptide employed, is considered appropriate. Continued therapy follows from the hypoestrogenic state initially established.

The drug formulations and routes of administration for delivery of LHRH or an agonist thereof and the progestational agent are well known in the art and include formulations for oral, nasal, intrabronchial, rectal, vaginal, parenteral (subcutaneous, intramuscular, intravenal, intraarterial, etc.) administration. In a preferred example, formulations for human use may provide about 50 to 1000 micrograms per day of LHRH or an agonist thereof (e.g. about 600 micrograms per day of $D-Trp^6-N^{\alpha}-MeLeu^7-Des-Gly^{10}-Pro^9-NHEt-LHRH$) and about 40 to 100 micrograms of progestational agent per day (e.g. about 50 micrograms per day of levonorgestrel). The formulations may be prepared for simultaneous or sequential use.

## CLAIMS

(for the contracting states:   CH   FR   GB ˙LI   LU   NL   SE)

1.  A composition of matter for the treatment of endometriosis in a female placental mammal comprising LHRH or an agonist thereof and a progestational agent, with the proviso that the progestational agent is other than medrogestone.

2.  A composition of matter as claimed in Claim 1 wherein the LHRH or agonist thereof is in an amount sufficient to produce a hypoestrogenic state in the female mammal and the progestational agent is in an amount to decidualize endometrial  tissue.

3.  A composition of matter as claimed in Claim 1 or 2 wherein the progestational agent is levonorgestrel, desogestrel or medroxyprogesterone acetate.

4.  A composition  as claimed in any one of Claims 1 to 3 wherein the LHRH agonist is $D\text{-}Trp^6\text{-}Des\text{-}Gly^{10}\text{-}Pro^9\text{-}NHEt\text{-}LHRH$, $D\text{-}Trp^6\text{-}N^\alpha\text{-}MeLeu^7\text{-}Des\text{-}Gly^{10}\text{-}Pro^9\text{-}NHEt\text{-}LHRH$ or $D\text{-}Ser(t\text{-}butyl)^6\text{-}Des\text{-}Gly^{10}\text{-}Pro^9\text{-}NHEt\text{-}LHRH$.

5.  A package for simultaneous or sequential use in the treatment of endometriosis in a female placental mammal which comprises a first pharmaceutical composition comprising LHRH or an agonist thereof and a pharmaceutically acceptable carrier and a second pharmaceutical composition comprising a progestational agent and a pharmaceutically acceptable carrier, with the proviso that the progestational agent is other than medrogestone.

6. A package as claimed in Claim 5 wherein the progestational agent is levonorgestrel, desogestrel or medroxyprogesterone acetate.

7. A package as claimed in Claim 5 or 6 wherein the LHRH agonist is D-Trp$^6$-Des-Gly$^{10}$-Pro$^9$-NHEt-LHRH, D-Trp$^6$-N$^\alpha$-MeLeu$^7$-Des-Gly$^{10}$-Pro$^9$-NHEt-LHRH or D-Ser(t-butyl)$^6$-Des-Gly$^{10}$-Pro$^9$-NHEt-LHRH.

8. The use in the treatment of endometriosis in a female placental mammal of LHRH or an agonist thereof in an amount and from a time sufficient to establish a hypoestrogenic state and of a progestational agent administered during the hypoestrogenic state in an amount and for a time to produce an atrophic nonendometrial implant.

9. The use as claimed in Claim 8 wherein the progestational agent is levonorgestrel, desogestrel or medroxyprogesterone acetate.

10. The use as claimed in Claim 8 or 9 wherein the LHRH agonist is D-Trp$^6$-Des-Gly$^{10}$-Pro$^9$-NHEt-LHRH, D-Trp$^6$-N$^\alpha$-MeLeu$^7$-Des-Gly$^{10}$-Pro$^9$-NHEt-LHRH or D-Ser(t-butyl)$^6$-Des-Gly$^{10}$-Pro$^9$-NHEt-LHRH.

CLAIMS
(for the contracting state: AT)

1. A process for preparing a composition of matter for the treatment of endometriosis in a female placental mammal which comprises bringing LHRH or an agonist thereof into association with a progestational agent, with the proviso that the progestational agent is other than medrogestone.

2. A process as claimed in Claim 1 wherein the LHRH or agonist thereof is in an amount sufficient to produce a hypoestrogenic state in the female mammal and the progestational agent is in an amount to decidualize endometrial tissue.

3. A process as claimed in Claim 1 or 2 wherein the progestational agent is levonorgestrel, desogestrel or medroxyprogesterone acetate.

4. A process as claimed in any one of Claims 1 to 3 wherein the LHRH agonist is $\text{D-Trp}^6\text{-Des-Gly}^{10}\text{-Pro}^9\text{-NHEt-LHRH}$, $\text{D-Trp}^6\text{-N}^\alpha\text{-MeLeu}^7\text{-Des-Gly}^{10}\text{-Pro}^9\text{-NHEt-LHRH}$ or $\text{D-Ser(t-butyl)}^6\text{-Des-Gly}^{10}\text{-Pro}^9\text{-NHEt-LHRH}$.

5. A process for preparing a package for simultaneous or sequential use in the treatment of endometriosis in a female placental mammal which comprises bringing a first pharmaceutical composition comprising LHRH or an agonist thereof and a pharmaceutically acceptable carrier into association with a second pharmaceutical composition comprising a progestational agent and a pharmaceutically acceptable carrier, with the proviso that the progestational agent is other than medrogestone.

6. A process as claimed in Claim 5 wherein the progestational agent is levonorgestrel, desogestrel or medroxyprogesterone acetate.

7. A process as claimed in Claim 5 or 6 wherein the LHRH agonist is D-Trp$^6$-Des-Gly$^{10}$-Pro$^9$-NHEt-LHRH, D-Trp$^6$-N$^\alpha$-MeLeu$^7$-Des-Gly$^{10}$-Pro$^9$-NHEt-LHRH or D-Ser(t-butyl)$^6$-Des-Gly$^{10}$-Pro$^9$-NHEt-LHRH.